# EUROPEAN PATENT APPLICATION

(11) **EP 0 842 646 A1**
(43) Date of publication of application: **20.05.1998**
(21) Application number: 97117808.2
(22) Date of filing: 15.10.1997
(51) Int. Cl.: A61F 5/08

(54) **Improved nasal dilator**

(30) Priority: 15.11.1996 IT MI962377
(71) Applicant: Also S.p.A., 22020 Zelbio (Como) (IT); Tecnoconvert S.p.A., 20020 Cornaredo (Milano) (IT)
(72) Inventor: Pinocchio, Massimo, 20153 Milano (IT)
(74) Representative: Cioni, Carlo

(57) **Abstract**

Dilator device of the nasal cavity of the type applied to the external surface of the nasal cavity characterized by comprising a strip of composite material comprising a first layer (1) of non-woven fabric, a layer (2) of plastic polymer material and a second layer (1') of non-woven fabric on which is deposited a biocompatible adhesive which is protected before use by a layer (3) of silicone paper or similar easily removed low-adhesion product.

## Description

The present invention relates to a new nasal dilator of a type applied externally.

In particular, the present invention relates to a device for preventing the walls of the nasal air-ducts from approaching each other or coming into contact during inspiration and reducing the volume of air breathed in and thus rendering breathing more difficult.

The problems of difficulty in breathing may result from disease, defects in the respiratory system or transitory effects and may develop during the exercise of most sports activities.

Nasal dilators are known which keep the nasal air-ducts free by exercising a force on the external surface of the same which tends to prevent the internal walls of the air-ducts from approaching the cartilaginous septum.

In particular, the Patent WO-92/22240 revealed devices comprising a flat strip of deformable material which is adaptable to the shape of the nose and which adheres simultaneously to the outer surface of both nasal air-ducts and of thinner strips of a resilient material fixed to the said deformable material which tends to make the flexible material assume its original flat structural form.

The devices described in the said application have the following drawback: that the strips of resilient material applied to the flexible material cause the device to project in their position which on the one hand form a grip which is a possible cause of accidental detachment of the device e.g. where either sunglasses or corrective glasses must be worn at the same time, and on the other hand is a source of unpleasant disturbance.

A device has been now discovered, and forms a primary objective of the present invention, which keeps the nasal air-ducts free but does not have the aforementioned drawbacks.

The device according to the present invention comprises a strip of composite material comprising a first layer of non-woven fabric, a layer of polymer material, and a second layer of non-woven fabric on which there is deposited a biocompatible adhesive which is protected before use by a layer of silicone paper or other low-adherence, easily removed material.

The device according to the present invention adheres with all its surface to the skin of the nose thus avoiding all risk of being detached. The nature of the non-woven fabric makes it feel very similar to a normal band-aid plaster with high tissue compatibility thus reducing to a minimum the sensation of the presence of a foreign body.

The non-woven fabric which comprise the first and third layers of the dilator device according to the present invention may be similar or different to each other, and are generally obtained by gluing fibres or micro-fibres of a textile polymer material. The non-woven fabric according to the present invention may be comprised of synthetic, artificial or natural fibres. Polyester, cellulose or other products normally used for the preparation of non-woven fabric may be used. In place of non-woven fabric, cotton or cellulose gauze may be used but this solution is less preferred.

The second layer of the device according to the present invention comprises a film of plastic polymer material such as polyester, polyolefine such as polyethylene or polypropylene, polycarbonate or similar material.

The layers of non-woven fabric may be glued to the layer of plastic polymer or they may be produced directly on the layer of plastic polymer.

The thickness of the device according to the invention is not critical. Advantageously, such thickness should be between 0.005 and 1.0mm, and preferably between 0.01 and 0.07mm.

The device according to the present invention may be obtained by cutting from a continuous roll of material comprising the first layer of non-woven fabric, the layer of plastic polymer and a second layer of non-woven fabric complete with the adhesive and protective paper.

The dilator device according to the present invention is substantially rectangular. It may, however, be given a shape more adapted to the surface to which it must be applied. This may be achieved by rounding the comers and/or making cutouts in the short side of the rectangle and/or by giving a curved shape to one or both of the longer sides.

An advantageous shape of the device according to the invention is that where the corners are rounded, two adjacent corners on the long side of the rectangle are extended to form lobes which are substantially larger than the other two rounded corners. Such a shape has been shown to be particularly suited to guaranteeing adhesion to the external surface of the nose.

The invention will now be described in more detail with reference to the attached drawing, it being understood that the detailed description and the drawing are as an example and must not be interpreted as limiting the scope of the invention.

Figure 1 shows an enlarged cross-section of the material which comprises the device according to the invention.

Figure 2 shows in plan a preferred shape of an embodiment of the device according to the invention.

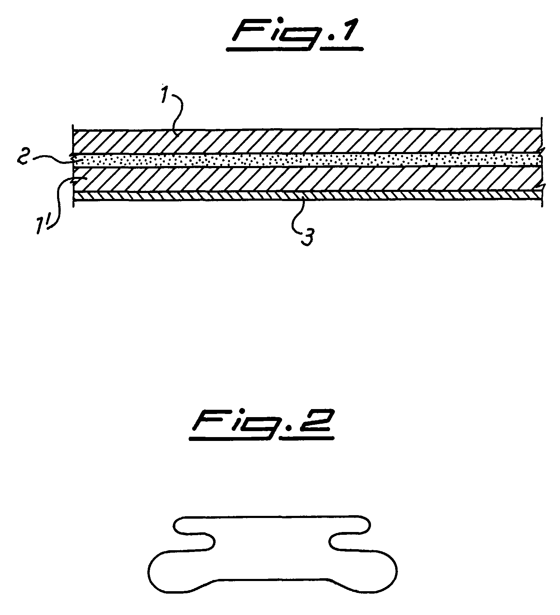

In Figure 1 the layers of non-woven fabric comprised of polyester fibres are shown by 1 and 1'. The layer of plastic material comprising a film of polyester is indicated by 2. The layer of protective silicone paper is shown as 3.

The material described in Figure 1 may form part of a continuous roll or may be produced in sheets depending on the apparatus used to cut out the device in its final form.

Figure 2 shows the finished device got by cutting out the material of Figure 1. The shape is rectangular with cut-outs in the shorter sides and with corners rounded, two of the corners on a long side are extended to form lobes substantially larger than the other two corners. Such a shape has been shown to be particularly suited to guaranteeing adhesion to the external surface of the nose.

The devices according to the invention may be different sizes to suit different shapes of nose.

## Claims

1. Dilator device of the nasal cavity of the type applied to the external surface of the nasal cavity characterized by comprising a strip of composite material comprising a first layer of non-woven fabric, a layer of plastic polymer material and a second layer of non-woven fabric on which is deposited a biocompatible adhesive which is protected before use by a layer of silicone paper or similar easily removed low-adhesion product.

2. Dilator device of the nasal cavity according to Claim 1 characterized by adhering on all its surface to the external nose epidermis thus avoiding all risk of detachment from the same.

3. Dilator device of the nasal cavity according to one or more of the preceding Claims characterized by the first and second non-woven fabric which comprise the material of the device, being of the same nature.

4. Dilator device of the nasal cavity according to one or more of the preceding Claims characterized by the shape being a long rectangle.

5. Dilator device of the nasal cavity according to the preceding Claim characterized by the corners being rounded.

6. Dilator device of the nasal cavity according to the preceding Claim characterized by the shorter side of the rectangle having cut-outs.

7. Dilator device of the nasal cavity according to the preceding Claim characterized by one or both the longer sides of the rectangle having a slightly curvilinear form.

8. Device according to one or more of the preceding Claims characterized by two of the adjacent corners on a long side being extended to form lobes which are substantially larger than the other two rounded corners.
